# EUROPEAN PATENT SPECIFICATION

(11) **EP 1 899 982 B1**
(45) Date of publication and mention of the grant of the patent: **08.07.2009**
(21) Application number: 06759924.1
(22) Date of filing: 12.05.2006
(51) Int. Cl.: G21F 5/018, A61M 5/178

(54) **RADIOPHARMACEUTICAL CONTAINER HAVING SYRINGE CAPPER**
RADIOPHARMAZEUTISCHER CONTAINER MIT INJEKTIONSNADEL-ENTKAPPUNGSVORRICHTUNG
CONTENANT RADIOPHARMACEUTIQUE MUNI D'UNE COIFFE DE LA SERINGUE

(30) Priority: 16.05.2005 US 681565 P
(43) Date of publication of application: 19.03.2008
(73) Proprietor: MALLINCKRODT, INC., St. Louis Missouri 63134 (US)
(72) Inventor: FAGO, Frank, M., Mason, Ohio 45040 (US)
(74) Representative: Chettle, Adrian John
(86) International application number: PCT/US2006/018905
(87) International publication number: WO 2006/124891

(56) References cited:
- WO-A-20/04036597
- US-A- 5 828 073
- US-A1- 2005 261 540

## Description

### FIELD OF INVENTION

This invention relates to systems and methods for reducing the likelihood of needle stick injuries, and more particularly, to systems and methods for reducing the risk of needle stick injuries to health care workers in the recapping of a hypodermic needle of a radiopharmaceutical syringe.

### BACKGROUND

The Needle Stick Prevention Act and the Occupational Safety and Health Administration (OSHA) regulations require organizations to take steps to reduce the likelihood of inadvertent needle stick injuries. Often syringes having needles are provided with plastic caps that cover the needle and inhibit needle stick injuries. The caps are removed before use, exposing the sharp needle. After use of the syringe, the needle must be disposed of in a way that protects people from being stuck by the needle and thereby potentially being exposed to blood borne pathogens. One way in which this could be accomplished is by replacing the cap over the used needle. However, the very act of holding the cap in one hand while guiding the needle into the cap with the other poses a danger of accidental needle stick injury to the hand holding the cap. Thus, recapping of a needle in this manner is generally undesirable.

Numerous safety syringes and disposal containers have been designed to reduce the risk of needle stick injuries. One type of disposal system involves a single container made of puncture resistant material that receives multiple spent syringes. Such a system has certain drawbacks. In the case where radiopharmaceuticals are used, unless the container is also a radiation shield it is unacceptable for receiving spent syringes containing residual radioactive radiopharmaceutical material. Containers with multiple spent syringes must be carefully designed to avoid presenting a sharps hazard upon disposal of a syringe into the container already containing numerous spent syringes. Other systems provide individual puncture resistant containers for the syringes. These require the manufacture of additional parts (i.e., the container) for each syringe and also must be located by the medical technician after use of the syringe. If the container is to be used with a radiopharmaceutical, it will have to be placed inside a radiation shield. The radiation shield (generally referred to as a "pig") may have to be made larger to accommodate the container and syringe.

WO-A-2004/036597 discloses a radiation shielding container having the features of the pre-characterising portion of claim 1 appended hereto.

### SUMMARY

One aspect of the invention is directed to a radiopharmaceutical container that is designed to at least generally promote safe recapping of radiopharmaceutical syringes. This radiopharmaceutical container is configured to accommodate (e.g., house) a radiopharmaceutical syringe including a needle and a syringe cap covering the needle. The radiopharmaceutical container provides radiation shielding for radiation emitted by a radiopharmaceutical in a radiopharmaceutical syringe. According to the invention there is provided a radiation-shielding container having the features of claim 1 appended hereto. For instance, in one characterization, the first portion may be referred to as a base, and the second portion may be referred to as a lid that may be releasably attached (e.g., screwed on, snapped on, friction-fitted on, or any other appropriate releasable attachment) to the base. The cap retainer holds the syringe cap so that the cap is held in a position in which an open end of the cap is oriented for insertion of the radiopharmaceutical syringe needle therein after use of the needle. For instance, the syringe cap and cap retainer may be designed to interface via a bayonet fit and/or high-helix threading to facilitate the selective holding of the syringe cap by the cap retainer. As another example, the syringe cap and cap retainer may be designed to allow for a snap-fit-type interface to facilitate the selective holding of the syringe cap by the cap retainer. Accordingly, the needle of the radiopharmaceutical syringe may be recapped without holding the syringe cap in a hand during insertion of the needle into the cap.

Another aspect of the invention is directed to a method for capping a radiopharmaceutical syringe having a hypodermic needle using at least a portion of a radiopharmaceutical syringe container (e.g., a pig). A syringe cap is engaged with a cap retainer associated with a portion of the radiopharmaceutical container so that the cap retainer holds the syringe cap. The radiopharmaceutical syringe is used to administer a radiopharmaceutical to a patient and thereby produce a spent radiopharmaceutical syringe. The hypodermic needle of the spent radiopharmaceutical syringe is inserted into the syringe cap while the syringe cap is held by the cap retainer to secure the syringe cap to the syringe. It is possible according to some protocols within this aspect of the invention to insert the needle of the spent radiopharmaceutical syringe into the syringe cap without manually holding either the radiopharmaceutical container or the syringe cap. This reduces the risk of needle stick injury.

Various refinements exist of the features noted in relation to the above-mentioned aspects, and further features may also be incorporated in the above-mentioned aspects, within the scope of the appended claims.

### BRIEF DESCRIPTION OF THE DRAWINGS

FIG. 1 is a perspective of a radiopharmaceutical container of the present invention;

FIG. 2 is a vertical section of the radiopharmaceutical container of Fig. 1;

FIG. 3 is a perspective of the radiopharmaceutical container of Fig. 1 being used to recap a needle on a spent radiopharmaceutical syringe;

FIG. 4 is a perspective of another radiopharmaceutical container of the present invention;

FIG. 5 is a perspective of a top portion of the radiopharmaceutical container of Fig. 4 including cap retainers;

FIG. 5a is a bottom plan view of the top portion;

FIG. 6 is a section of the top portion taken in the plane including line 6-6 of FIG. 5a;

FIG. 7a is a schematic of a radiopharmaceutical container top portion including a cap retainer that is operable to at least temporarily grip (e.g., substantially immobilize) a radiopharmaceutical syringe cap;

FIG. 7b is a fragmentary section taken in the plane including line 7b-7b of FIG. 7a;

FIG. 8a is a schematic diagram of another cap retainer that is operable to at least temporarily grip a radiopharmaceutical syringe cap;

FIG. 8b is a section taken in the plane including line 8b-8b of Fig. 8a;

FIG. 8c is an enlarged detail of Fig. 8b;

FIG. 9 is a schematic fragmentary vertical section of another syringe cap and radiopharmaceutical container including a cap retainer operable to at least temporarily grip a syringe cap; and

FIG. 10A-10D show a sequence of operation of the radiopharmaceutical syringe cap retainer of Fig. 9.

Corresponding reference characters indicate corresponding parts throughout the drawings.

### DETAILED DESCRIPTION

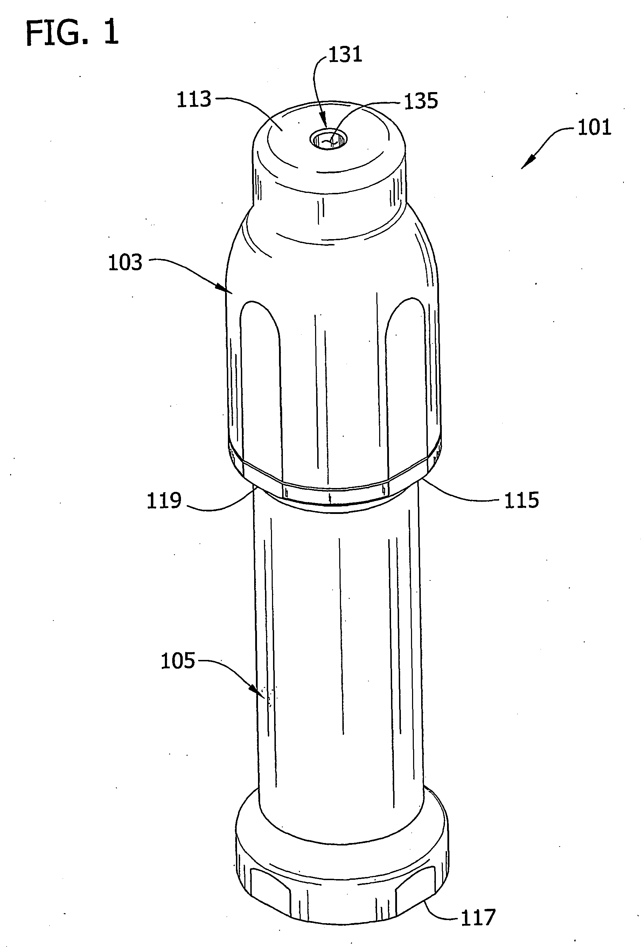

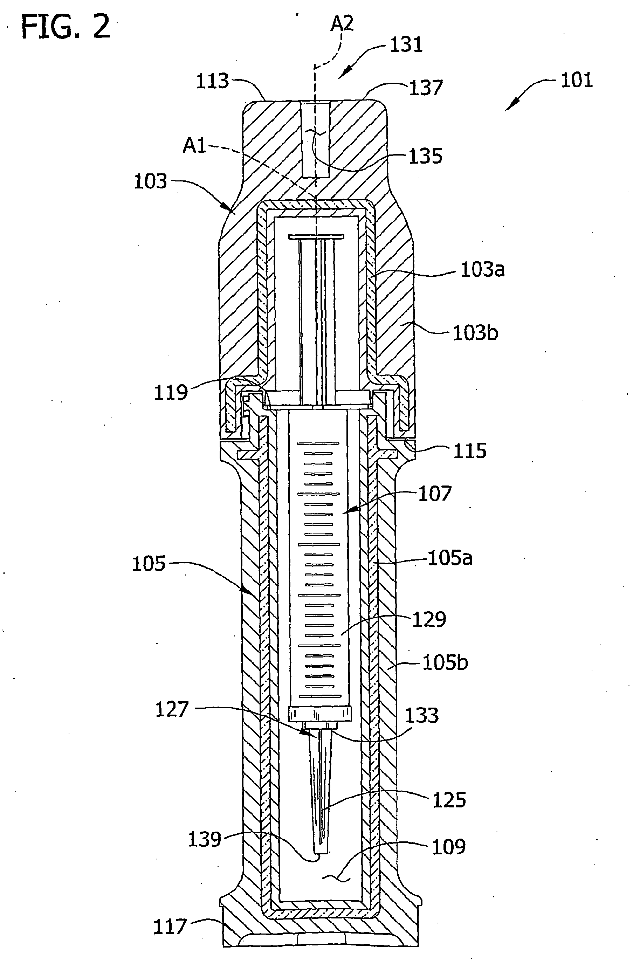

Referring to the drawings, Figs. 1-3 illustrate a radiopharmaceutical container generally designated 101. This type of container is known as a radiopharmaceutical pig. Except as described hereinafter, the pig 101 can be of any appropriate radiation-shielding construction. The radiopharmaceutical pig 101 includes top and bottom (e.g., "first" and "second") portions 103, 105 that are releasably securable to one another for enclosing a radiopharmaceutical syringe 107 in a cavity 109 (Fig. 2). A radiopharmaceutical container may have a different number and/or different arrangement of component parts within the scope of the present invention. As shown in the drawings, for example, the top portion 103 has a generally tubular shape with a closed top end 113 and an open bottom end 115. The bottom portion 105 has a generally tubular shape with a closed bottom end 117 and an open top end 119. When the two portions 103, 105 are secured to one another, the open bottom end 115 of the top portion 103 aligns with the open top end 119 of the bottom portion 105 so that the top and bottom portions together define the cavity 109 for enclosing the radiopharmaceutical syringe 107. The radiopharmaceutical syringe 107 shown in the drawings contains a dose of a liquid radiopharmaceutical. The radiopharmaceutical pig 101 is generally used to shield the surrounding environment from radiation (e.g., during transport of radiopharmaceuticals). The top and bottom portions 103, 105 of the radiopharmaceutical pig 101 include one or more shielding materials (e.g., lead, tungsten, depleted uranium, tungsten impregnated plastic) and are capable of substantially shielding the surroundings from radiation emitted by the radiopharmaceutical when the syringe 107 is enclosed in the pig 101. For example, the top and bottom portions 103, 105 may include shielding elements 103a, 105a enclosed in polymer shells 103b, 105b as discussed in more detail in co-owned U.S. Patent Application Serial No. 10/527,301 (published as U.S. Pat. Pub. No. 20050224730). However, pigs having other constructions, including (but not limited to) those that are constructed of solid shielding material, those that have shielding materials enclosed in a shell (e.g., a polymer shell), those having a liner (e.g., a plastic liner) lining the inside of the shield materials, and those that have shielding materials distributed substantially evenly through their bodies are also within the scope of the invention.

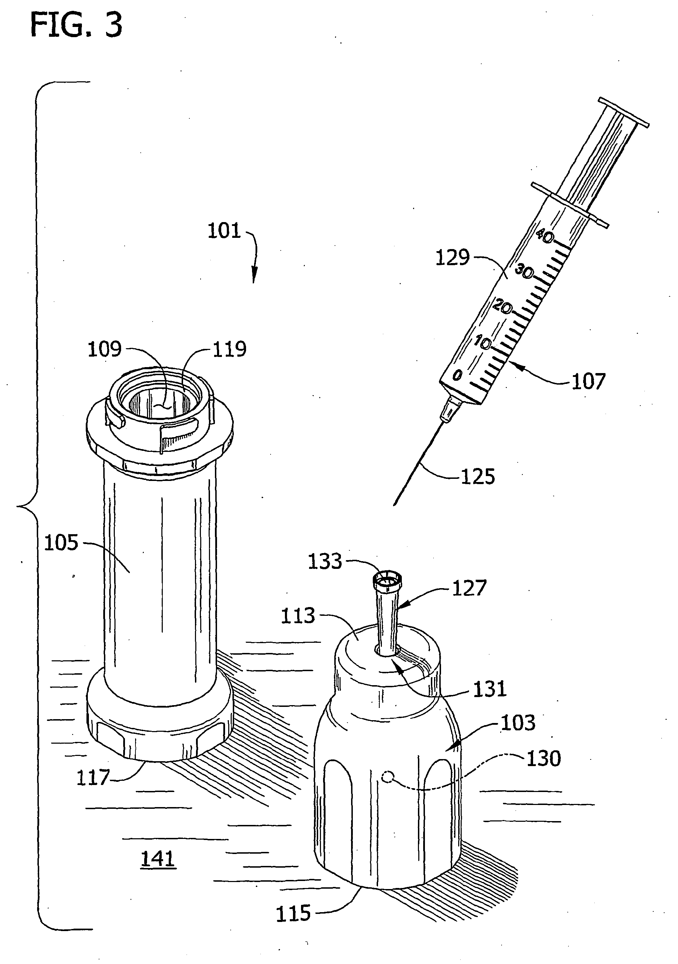

The radiopharmaceutical syringe 107 shown in Fig. 2 is equipped with a hypodermic needle 125 attached to the syringe. The hypodermic needle 125 is shown in Fig. 2 as being enclosed within a syringe cap 127 (e.g., a protective sheath designed to cover at least the tip of the needle 125) fastened to the body 129 of the syringe 107. As shown in the drawings, the syringe cap 127 is transparent, but the cap 127 could be translucent, opaque or any combination of transparent, translucent, and opaque without departing from the scope of the invention. The syringe cap 127 of Figs. 1-3 is of conventional construction. The radiopharmaceutical pig 101 also includes a cap retainer 131 to accommodate at least a portion of the syringe cap 127 and to at least generally hold the cap in a position in which an open end 133 of the cap is disposed for re-insertion of the syringe needle 125 therein. Thus, the cap retainer 131 may be used in accordance with methods described later herein to facilitate recapping of the needle 125 with the syringe cap 127 (e.g., after use of the needle) without holding the syringe cap in a hand during insertion of the needle into the cap. The cap retainer 131 is shown as a cavity 135 defined in an exterior surface 137 of the top portion 103 of the pig 101. The cap retainer 131 is sized and shaped to receive a closed end part 139 of the syringe cap 127, as shown in Fig. 3 for example. When the closed end part 139 of the syringe cap 127 is disposed in the cavity 135, the cap retainer 131 at least generally holds the cap so that it projects outwardly from the top end 113 of the radiopharmaceutical pig 101 and presents an open end part 133 of the syringe cap for receiving the needle 125 attached to the radiopharmaceutical syringe 107. In the embodiment shown in the drawings, the cap retainer 131 is centrally located in the top end 113 of the top portion 103 of the radiopharmaceutical pig 101, but the cap retainer can be located.elsewhere without departing from the scope of the invention. For example, a cap retainer of another embodiment may be a separate component that may be attached to a radiopharmaceutical pig rather than being integral with the pig. The cavity 135 shown in the drawings is a frustoconical cavity. However, a cap retainer can suitably by formed by a cavity having a different shape, including a substantially cylindrical cavity, a channel or groove shaped cavity (e.g., a channel having a C-shaped cross section) open at one or both ends, and many others.

The cap retainer 131 may be supported by a free standing portion of the radiopharmaceutical pig 101 (i.e., a portion of the pig that is able to retain itself independently in a stable position on a support surface) so that the cap retainer is held in position to receive syringe cap 127 by the freestanding portion. This provides a desirable alternative to manually holding the radiopharmaceutical pig 101 during recapping. For example, as shown in Fig. 3, the cap retainer 131 may be supported by the top portion 103 of the pig 101. The top portion 103 of the radiopharmaceutical pig 101, in turn, may be adapted to hold the cap retainer 131 in a particular orientation in which the open end 133 of the retainer is accessible to users (e.g., open end 133 up) when it is placed on a surface such as a table top. Further the freestanding portion of the radiopharmaceutical pig 101 that supports the cap retainer 131 may be constructed in a manner that facilitates recapping of the needle without manually holding the pig. In the illustrated embodiment, for example, the weight of the top portion 103 of the pig 101 may exceed a securement force required to secure the syringe cap 127 to the radiopharmaceutical syringe 107. Similarly, the top portion 103 of the radiopharmaceutical pig 101 may have a total mass and center of gravity 130 (shown schematically in Fig. 3) located so the force needed to tip the freestanding portion of the pig over is greater than the force needed to secure the syringe cap 127 to the radiopharmaceutical syringe 107. Likewise, the top portion 103 of the radiopharmaceutical pig 101 may be constructed to hold the cap retainer in an orientation (e.g., inclined or vertical as shown in Fig. 3) so that a substantial component of the securement force is applied in a direction normal to the support surface. For example, the freestanding portion of the radiopharmaceutical pig 101 (e.g., the top portion 103) may have a longitudinal axis A1 and the cap retainer 131 may have a longitudinal axis A2 generally parallel to (e.g., coincident with) the longitudinal axis of the container, as shown in Fig. 2. These features make the top portion 103 resistant to being tipped over or slid along the support surface by forces applied during recapping, thereby facilitating recapping without manually holding the top portion during the recapping. Although the cap retainer 131 is supported by the top portion 103 of the radiopharmaceutical pig 101 in the illustrated embodiment, it is understood that a cap retainer could be supported by other freestanding container portions without departing from the scope of the invention. In some embodiments, the surface of the top portion 103 of the radiopharmaceutical pig 101 that is designed to interface with the support surface (e.g., table) may include any of a number of appropriate gripping or "anti-skid" features to prevent sliding of the top portion during recapping. For instance, that surface of the top portion 103 may be textured and or may include (e.g., be coated with) an appropriate gripping or "anti-skid" material.

One protocol for using the radiopharmaceutical pig 101 includes loading a dose of a radiopharmaceutical into the syringe 107. At least the sharp tip of the needle 125 may be enclosed in a syringe cap 127. The radiopharmaceutical syringe 107 containing the dose of the radiopharmaceutical may be enclosed in the cavity 109 of the pig 101 by securing the top and bottom portions 103, 105 together to engage a bayonet connection as is known in the art. The radiopharmaceutical pig 101 and the loaded radiopharmaceutical syringe 107 enclosed therein may be transported to a healthcare facility. The pig 101 shields the surrounding environment from radiation emitted by the radiopharmaceutical (e.g., during transport).

At the healthcare facility, the radiopharmaceutical pig 101 may be opened to remove the radiopharmaceutical syringe 107 therefrom. The top portion 103 of the pig 101 may be placed on a surface 141 (e.g., a table top surface), open end 115 down. The syringe cap 127 may be removed from the radiopharmaceutical syringe 107, and the closed end part 139 of the syringe cap 127 may be engaged with (e.g., inserted into the cavity 135 of) the cap retainer 131 on the closed top end 113 of the top portion 103 of the pig 101. The closed end part 139 of the syringe cap 127 could instead be inserted into the cavity 135 of the cap retainer 131 while the syringe cap 127 is attached to the radiopharmaceutical syringe 107. Accordingly, the cap retainer may be used to assist in removal of the syringe cap 127 from the radiopharmaceutical syringe 107. This cap retainer 131 is designed to hold the syringe cap 127 in a position in which the open end 133 of the syringe cap 127 is presented for receiving the tip of the needle 125 therein.

The radiopharmaceutical syringe 107 is used to administer the radiopharmaceutical to a patient, typically by subcutaneous injection, thereby resulting in the syringe exhibiting what is commonly referred to as a "spent" condition. Thereafter, the hypodermic needle 125 is potentially contaminated with blood borne pathogens and must be handled in accordance with applicable regulations and industry safety standards. These regulations and standards prohibit recapping of the needle 125 with the syringe cap 127 by holding the cap 127 in a hand because of the risk of a needle stick injury caused by the act of recapping. With the illustrated embodiments of the present invention, however, the syringe cap 127 is held by the cap retainer 131 rather than a human hand. Accordingly, while holding the spent syringe 107 in one hand, a healthcare worker can manually insert the tip of the hypodermic needle 125 attached to the spent radiopharmaceutical syringe into the syringe cap 127 and secure the syringe cap to the syringe without holding the syringe cap 127 in his or her other hand during insertion of the needle 125 into the cap 127. Thus, the other hand is not disposed along the path of movement of the sharp end of the needle 125 as it is moved toward the syringe cap 127. Further, if desired, the cap retainer 131 can be supported during recapping by a freestanding portion of the radiopharmaceutical pig 101 (e.g., the freestanding upper portion 103) without any manual holding of the pig. Thus, the recapping may be accomplished manually with only one hand.

Thereafter, the capped spent radiopharmaceutical syringe 107 may be re-enclosed in the cavity 109 of the radiopharmaceutical pig 101 and transported to a disposal facility that is equipped to handle waste that is likely both radioactive (e.g., from radiopharmaceutical residue in the syringe) and biologically contaminated (e.g., from blood residue associated with the needle 125 attached to the spent syringe). Other protocols may include uncapping the needle 125 without the use of the radiopharmaceutical pig 101 and utilizing the cap retainer 131 of the radiopharmaceutical pig during recapping.

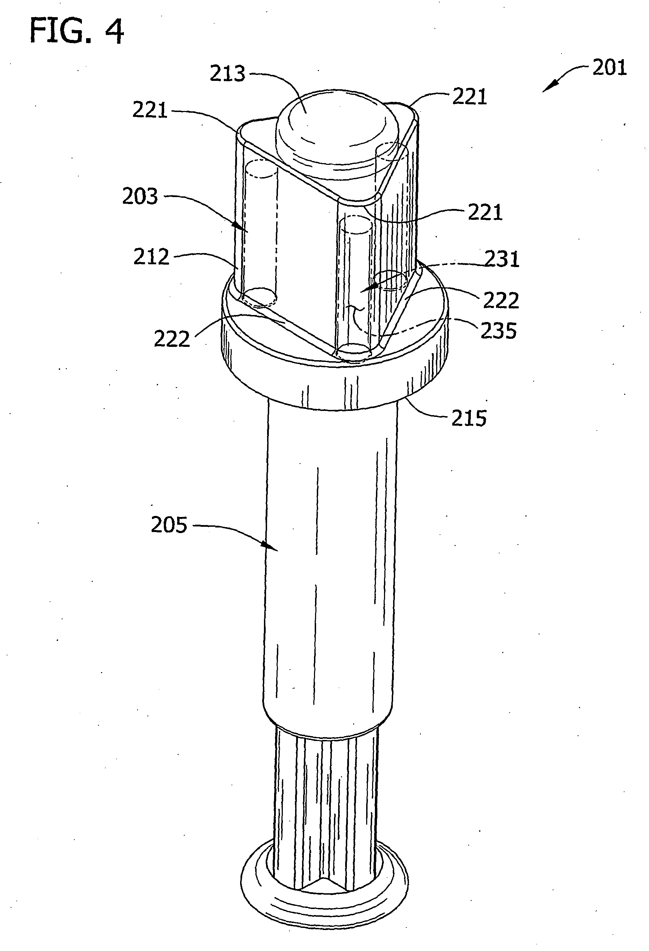

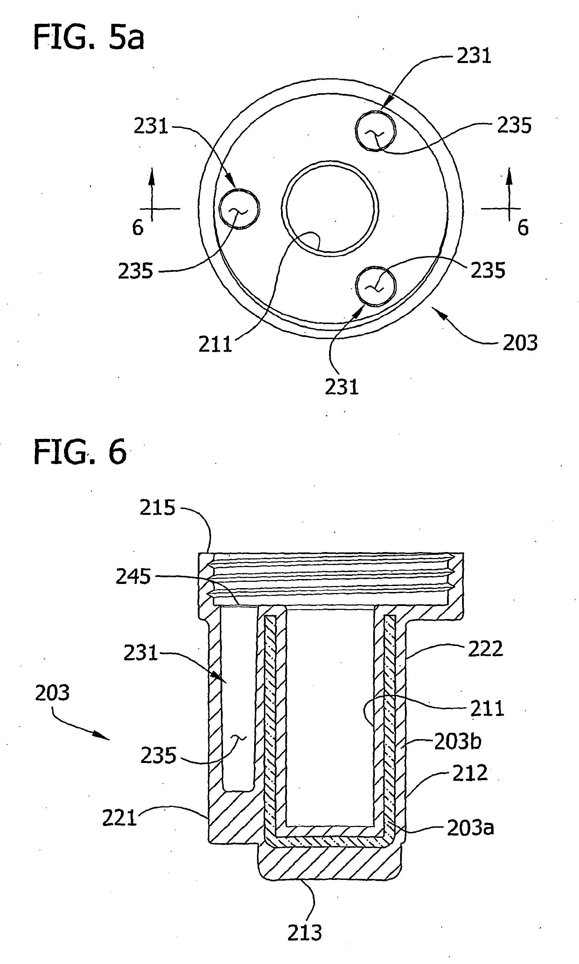

Another embodiment of a radiopharmaceutical pig, generally designated 201, is shown in Figs. 4-6. The radiopharmaceutical pig 201 has top and bottom portions 203, 205 that are releasably securable to one another for enclosing a radiopharmaceutical syringe 107 containing a dose of radiopharmaceutical in substantially the same manner as the radiopharmaceutical pig 101 shown in Figs. 1-3. The top and bottom portions 203, 205 include shielding materials, in the same manner as the pig 101 described above. For example, the top portion 203 shown in Fig. 6 includes a shielding element 203a enclosed in a shell 203b. The top portion 203 may be said to be somewhat similar to the top portion 103 shown in Figs. 1-3 in that it has a generally tubular shape with a closed top end 213 and an open bottom end 215. The top portion 203 has an interior sidewall 211 that is generally circular in cross section. However, unlike the top portion 103, the exterior sidewall 212 of the top portion 203 exhibits the general shape of a triangular prism. The top portion 203 of the pig 201 is significantly thicker at the vertices 221 of the prism than at the midpoints 222 between the vertices.

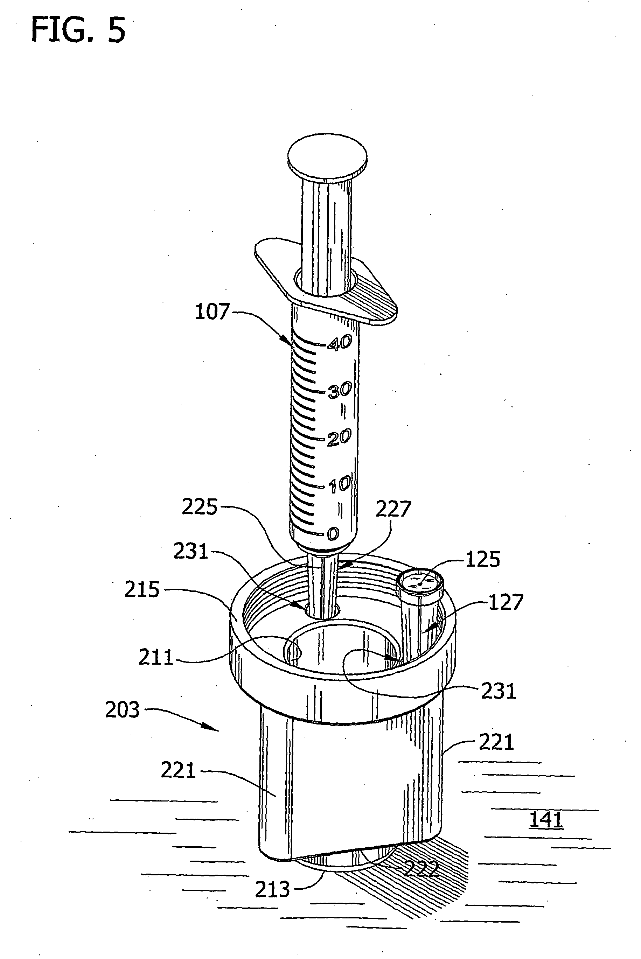

The radiopharmaceutical pig 201 also comprises a plurality (e.g., at least two) of cap retainers 231. Each cap retainer 231 is similar to the cap retainer 131 shown in Figs. 1-3 except for its location. For example, the radiopharmaceutical pig 201 shown in the drawings has three cap retainers 231 (Fig. 5a) spaced radially about the open end 215 of the top portion 203. As shown in Fig. 6, each cap retainer 231 comprises a cavity 235 defined in the top portion 203. The cavities 235 are located at respective vertices 221 of the prismatic exterior 212 so that the cavities are formed where the top portion 203 is thicker, thereby alleviating concern about reduced radiation shielding as a result of the cavities.

The cavities 235 are sized and shaped for receiving the closed end 139 of the syringe cap 127. Open ends 245 of the cavities 235, one of which is shown in Fig. 6, face the bottom portion 205 of the radiopharmaceutical pig 201 when the top portion 203 is secured to the bottom portion. Moreover, when the top and bottom portions 203, 205 of the embodiment of the pig 201 shown in Fig. 6 are secured to one another, the open ends 245 of the cavities 235 are enclosed therein. In other embodiments, however, the cavities (e.g., frustoconical and/or channel shaped cavities) of the cap retainers may be positioned at one or more of the vertices 221 of the prismatic exterior 212 so their open ends are accessible from the exterior of the pig 210 when the top and bottom portions 203, 205 are secured to one another.

The top portion 204 of the radiopharmaceutical pig 201 is capable of freestanding and is designed to support the cap retainers 231 in substantially the same manner as the top portion 103 of containers 101 (e.g., open end 245 of the cavity 235 facing up).

The radiopharmaceutical pig 201 operates in substantially the same way as radiopharmaceutical pig 101, except as noted herein. When the radiopharmaceutical pig 201 is opened, its top portion 203 is placed on a surface 141 with its closed top end 213 down. In this position, the open ends 245 of the cavities 235 of the cap retainers 231 may be said to face up, thereby facilitating access to the open ends of the cap retainers. Incidentally, the surface of the closed top end 213 of the top portion 203 that interfaces with the surface 141 is shown as being smaller in size/dimension than the opposing open end 215. It should be noted that that surface of the closed top end 213 of the top portion 203 that interfaces with the surface 141 may be larger or substantially equal in size/dimension to the opposing open end 215 in other embodiments. One of the cap retainers 231 may be used cap to the needle 125 attached to the spent syringe 107 in substantially the same manner as the cap retainer 131 of pig 101. If any radioactive or biologically contaminated fluid leaks from the radiopharmaceutical syringe 107 while it is being capped or uncapped, the fluid may fall into the top portion 203 of the pig 201 and may be enclosed in the radiopharmaceutical pig 201 when the top and bottom portions 203, 205 are reassembled for transport to a disposal facility. If this happens, the fluids are safely contained in the radiopharmaceutical pig 201 during transport to the disposed facility.

In another capping protocol, a plurality of cap retainers 231 may be used to facilitate switching needles on the radiopharmaceutical syringe 107. For example, it is not uncommon for the needle used to load the syringe to be larger than desired for injecting the radiopharmaceutical into a patient. A larger gauge needle, in most cases, generally includes a larger bore that facilitates loading of the radiopharmaceutical syringe with the radiopharmaceutical, but some patients may generally prefer to be injected with a smaller gauge needle. Thus, the needle 125, in at least some embodiments, may be characterized as a loading needle (e.g., a larger needle). The syringe cap 127 may be placed over the larger loading needle 125 before it is enclosed in the radiopharmaceutical pig 201 (e.g., for transport to a healthcare facility).

The radiopharmaceutical pig 201 may be opened (e.g., at the healthcare facility) and the radiopharmaceutical syringe 107 removed therefrom. Referring to Fig. 5a, the syringe cap 127 enclosing the loading needle 125 may be engaged with one of the cap retainers 231. The syringe cap 127 and the loading needle 125 enclosed therein may then be removed from the syringe 107. For example, the syringe cap 127 and loading needle 125 may be twisted relative to the syringe 107 while pulling the syringe cap 127 away from the syringe (e.g., using any of the gripping features described later herein) to remove the loading needle 125 and syringe cap 127 from the syringe 107. The cap retainer 231 of the radiopharmaceutical pig 201 may be designed to facilitate this removal of the loading needle 125 (or other needle for that matter) and associated syringe cap 127. For instance, the syringe cap 127 (having the loading needle 125 disposed therein) may be engaged with the cap retainer 231 of the radiopharmaceutical pig 201. The syringe 107 may be twisted or otherwise manipulated (depending on the particular type of engagements between the syringe cap 127 and the cap retainer 231, the syringe cap 127 and the needle 125, and the needle 125 and the radiopharmaceutical syringe 107) to dissociate the syringe cap 127 and needle 125 from the syringe 107.

A smaller gauge injection needle 225 may be attached to the radiopharmaceutical syringe 107 for injection of the radiopharmaceutical into a patient. As shown in Fig. 5, for example, a second syringe cap 227 enclosing the injection needle 225 may be engaged with another of the cap retainers 231 which holds the injection needle 225 and the second syringe cap 227 while one or both are being attached to the syringe 107. The injection needle 225 could be manually attached to the radiopharmaceutical syringe 107, either with or without the second syringe cap 227, without departing from the scope of the invention. The injection needle 225 may be used to inject the radiopharmaceutical into a patient, thereby resulting in a spent radiopharmaceutical syringe. The second syringe cap 227 may be engaged with one of the cap retainers 231 (e.g., left in the cap retainer) to cap the injection needle 225 of the spent syringe 107 without holding the second syringe cap 227 in a hand during insertion of the needle into the cap. Further, if desired, the respective cap retainer 231 may be supported by the freestanding portion of the radiopharmaceutical pig 201 (i.e., the freestanding upper portion 203) without manually holding the pig during the capping of the injection needle 225. The loading needle 125 and its syringe cap 127 may be removed from the respective cap retainer 231 and placed in a radioactive waste container at any time after they are detached from the radiopharmaceutical syringe 107. Those skilled in the art will appreciate the convenience afforded by use of multiple cap retainers on the same radiopharmaceutical pig, which allow the loading needle and its syringe cap to be held by a respective cap retainer while another cap retainer is being used in connection with the syringe cap for the injection needle. Further, if one of the cap retainers is damaged (e.g., if it has a gripping mechanism, as discussed below, that becomes damaged), the other cap retainers may be used without replacing or repairing the radiopharmaceutical pig.

In some radiopharmaceutical containers of the invention, the cap retainer does not actively grip the syringe cap. Instead, the syringe cap is gravitationally held by the cap retainer, which is an otherwise passive receptacle (e.g., a receptacle that may loosely, or at least may not tightly, interface with the needle cap), during capping of the needle of a spent syringe. In some cases, however, it will be desirable for the cap retainer to actively grip the syringe cap for at least part of the process. Those skilled in the art will recognize that numerous features could be modified and/or added to a cap retainer to allow the cap retainer to grip a syringe cap. Some of these features will now be described.

A depth of the cap retainers 231 may be any desired depth. For instance, in some embodiment, the depth of at least some of the cap retainers 231 is sufficient to enable the top and bottom portions 203, 205 of the radiopharmaceutical pig 201 to be secured together (e.g., to house a syringe inside) regardless of whether one or more syringe caps (e.g., 127, 227) are disposed in the cap retainer(s) 231. Accordingly, the cap retainers 231 of some embodiments may be designed to accommodate a substantial entirety of a syringe cap 127. In other words, some embodiments may allow a substantial entirety of the syringe cap 127 to fit within the cap retainer 231. Other embodiments may be designed to accommodate only a portion of a syringe cap 127 while still allowing the top and bottom portions 203, 205 of the pig 201 to be fitted together to form an enclosure. '

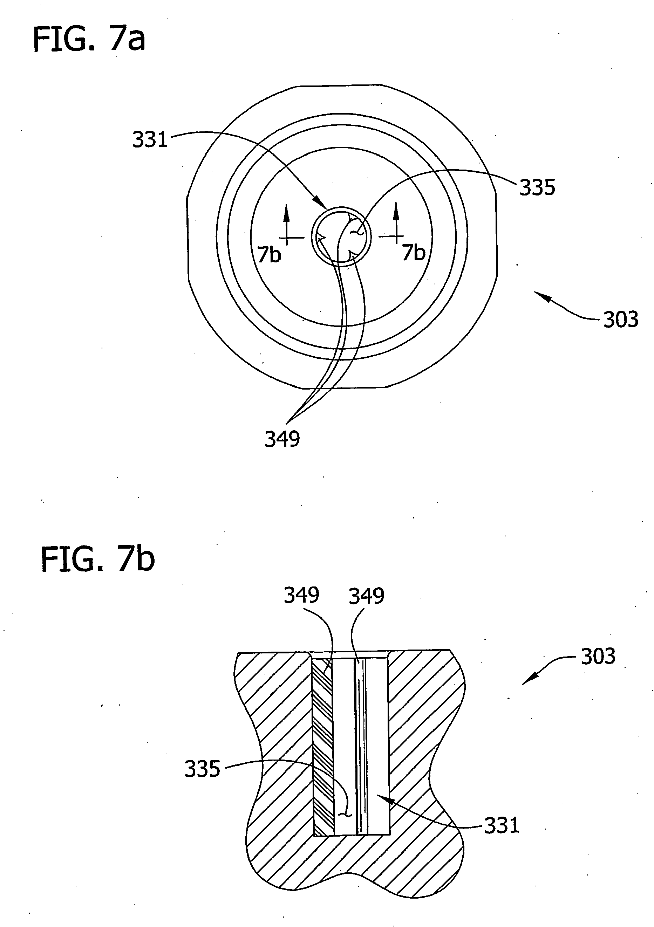

One way to provide for gripping of a syringe cap by a cap retainer may be to put one or more resilient projections (e.g., fingers and/or fins) in the cavity to create a friction fit when the closed end part of a syringe cap is engaged with the cap retainer. Referring to Figs. 7a and 7b, for example, a radiopharmaceutical pig top portion 303 includes a cap retainer 331 having three resilient fins 349 that are radially spaced about the inside of the cavity 335. As shown in the drawings, the fins 349 are integrally molded with the top portion 303. The fins could instead be part of an insert that is formed separately from the radiopharmaceutical pig and inserted in the cavity of the cap retainer. The insert could be secured in the cavity by any suitable means (e.g., adhesives, fasteners, and/or by using an insert having an interference fit with the side of the cavity). When the closed end part 139 of the syringe cap 127 (not shown in Figs. 7a and 7b) engages the cap retainer 331, the resilient fins 349 are deformed to allow the syringe cap to pass into the cap retainer. Because they are resilient and the syringe cap 127 widens away from the closed end 139, the fins 349 may be said to push against the syringe cap, thereby increasing friction between the syringe cap and the cap retainer 331 as the cap is pushed farther into the cavity 335.

A radiopharmaceutical pig may be manufactured with one more cap retainers 331 of the type shown in Figs. 7a and 7b. In one capping protocol of the invention, the syringe cap 127 may be engaged with the cap retainer 331 before use of the radiopharmaceutical syringe 107, allowing the cap retainer to grip the syringe cap 127. Then the cap retainer 331 may be used to grip the syringe cap 127 while the unused needle 125 is moved out of the cap by a user pulling on the radiopharmaceutical syringe 107. After injection of the radiopharmaceutical, the needle 125 of the now spent radiopharmaceutical syringe 107 may be recapped by inserting the needle into the open end 133 of the syringe cap 127 while it is held by the cap retainer 331. Once the radiopharmaceutical syringe 107 is recapped, the syringe cap 127 may be released from the cap retainer 331. For example, the user can grab the syringe cap 127 and pull it out of the cap retainer 331. In some cases, the syringe cap 127 snaps onto the radiopharmaceutical syringe 107 and can be pulled out of the cap retainer 331 by pulling on the syringe.

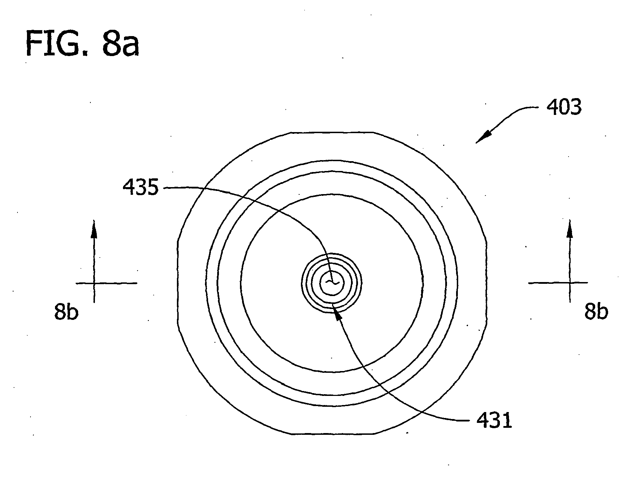

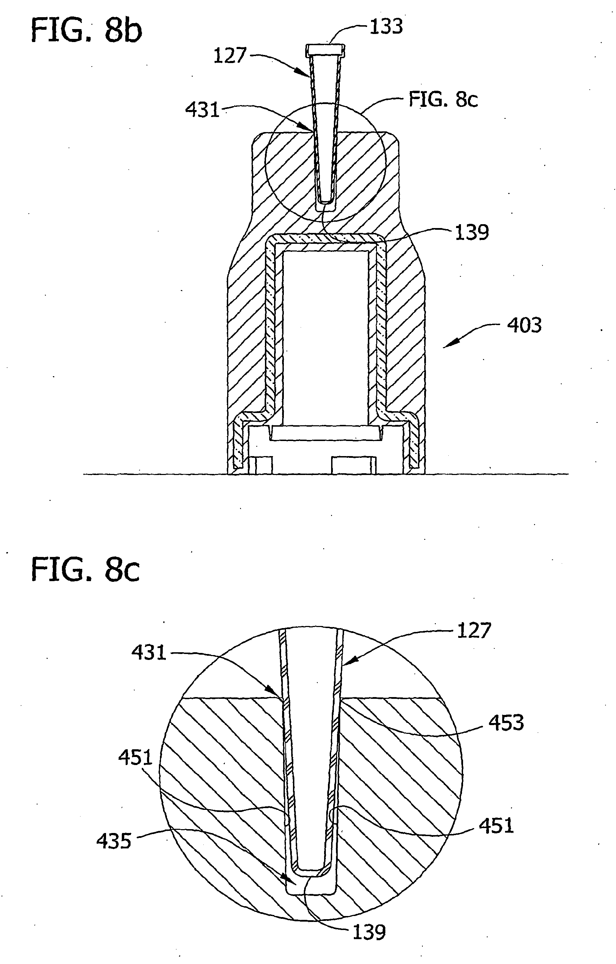

A top portion 403 of a radiopharmaceutical pig having a cap retainer 431 is shown in Figs. 8a-8c. This cap retainer 431 employs a tapered friction fit to grip the syringe cap 127. The syringe cap 127 is tapered from a larger diameter at the open end 133 of the syringe cap to a smaller diameter at the closed end part 139 of the syringe cap. The side 451 of the cavity 435 shown in Figs. 8a-8c is not tapered as much as the syringe cap 127. Consequently, the syringe cap 127 can only be inserted a limited extent into the cavity 435 before further movement into the cavity is resisted by interference between the tapered syringe cap 127 and the radiopharmaceutical pig at the open end 453 of the cavity 435. The syringe cap 127 may be made from any appropriate needle cap material (e.g., resilient material, such as various plastics). Thus, when the syringe cap is pressed into the cavity, a friction fit may be formed between the syringe cap and the cap retainer as walls of the cavity interface with the side of the syringe cap.

The cap retainer 431 shown in Figs. 8a-8c can be used in substantially the same manner as the cap retainer 331 shown in Figs. 7a-7b. One exception may be that the user inserts the closed end part 139 of a syringe cap 127 far enough into the cavity 435 to form a friction fit by interaction of the radiopharmaceutical pig and the tapered syringe cap 127.

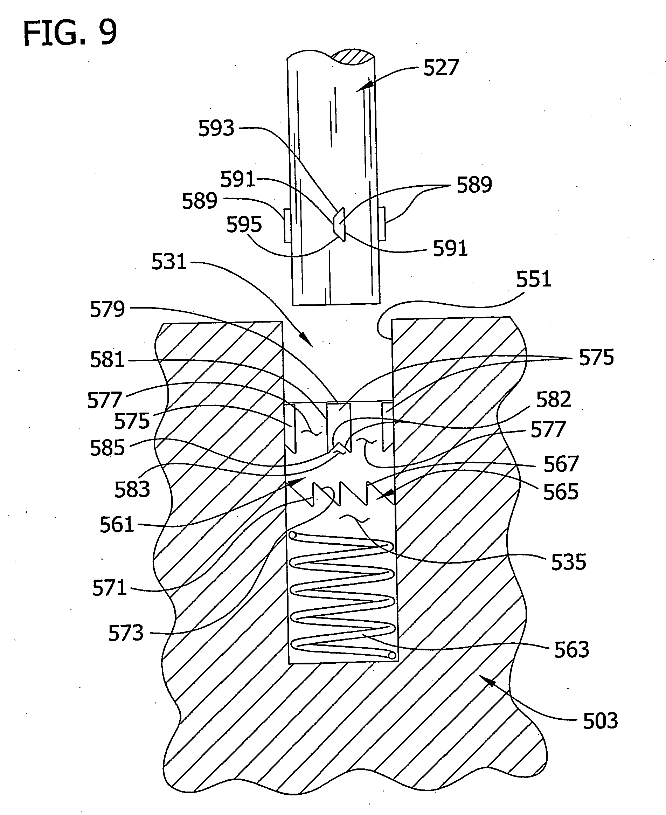

It is also possible to construct cap retainers with a spring-biased, selective release mechanism. For example, Fig. 9 is a schematic fragment of the top portion 503 of a radiopharmaceutical pig having a cap retainer 531 having a spring-biased release mechanism 561. Some will recognize the release mechanism 561 as being similar to a common actuating mechanism for ball point pens.

A spring 563 is disposed in the cavity 535 so that insertion of a syringe cap 527 into the cavity results in compression of the spring. Thus, the spring 563 is operable to bias the syringe cap 527 toward moving out of the cavity 535. A circumferentially extending shoulder 565 having a series of saw-tooth projections 567 thereon is provided on the inside wall 551 of the cavity 535. Each saw-tooth projection 567 has a vertical face 571 and an inclined face 573. A series of latch tabs 575 project inwardly from the inside wall 551 of the cavity 535. The latch tabs 575 are spaced above the shoulder 565: The latch tabs 575 are also spaced circumferentially from one another to define a plurality of channels 577 therebetween. Each latch tab 575 preferably has a horizontal top 579, parallel vertical sides 581, and two inclined lower faces 582 defining a notch 583 extending between the two lower corners 585 of the latch tabs.

The cap retainer 531 is designed to operate in cooperation with the syringe cap 527 shown in Fig. 9, which is preferably substantial the same the syringe cap 127 described above except that it has at least one, and preferably a plurality, of nubs 589 projecting outwardly from the side of the syringe cap. As shown in Fig. 9, for example, a series of four nubs 589 (three of which are visible) are circumferentially spaced from one another a distance corresponding to the distance (or a multiple thereof) between adjoining channels 577 in the cap retainer 531. The nubs 589 preferably have parallel vertical sides 591, one long and one short, and inclined upper and lower surfaces 593, 595. The inclination of the upper surface 593 of each nub 589 is in a direction opposite the inclination of the lower surface 595 thereof. The nubs 589 shown in Fig. 9, for example, have an upper surface 593 that slopes up moving from left to right and a lower surface 595 that slopes down moving from left to right.

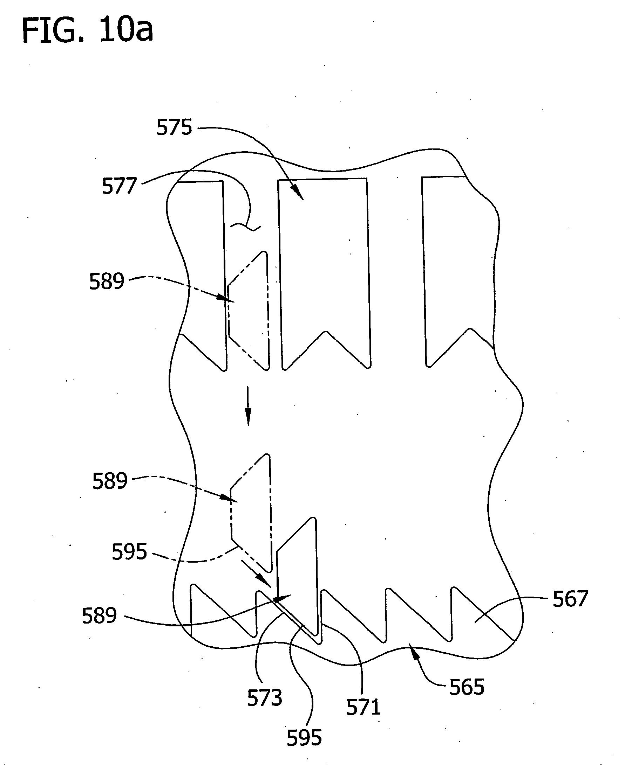

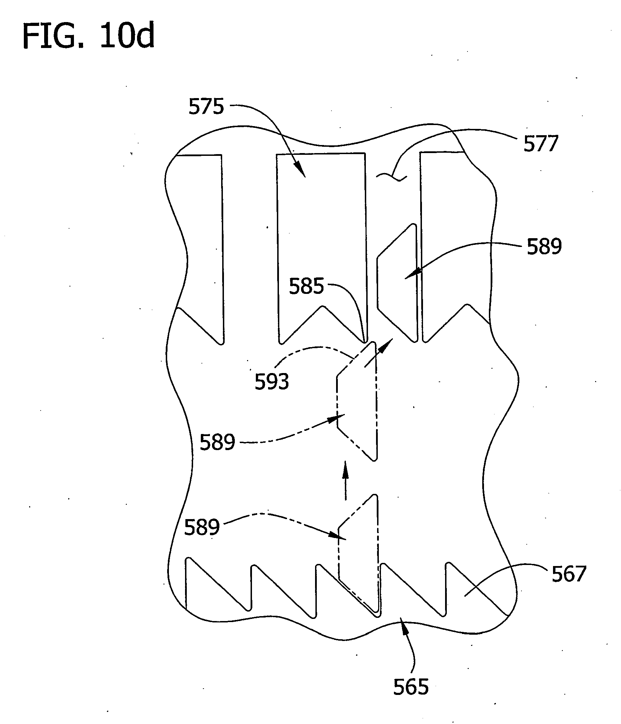

Referring now to Fig. 10a, when a user inserts the syringe cap 527 into the cap retainer, the nubs 589 pass between the latch tabs 575 through the channels 577. The insertion of the syringe cap 527 into the cavity 535 compresses the spring 563, which thereafter urges the syringe cap to move out of the cavity. As the syringe cap 527 is inserted farther into the cavity 535 by the user, the nubs 589 engage the inclined surfaces 573 of the saw-toothed projections 567 that are aligned with the channels 577. The saw-toothed projections 567 exert a torque on the syringe cap 527 as it is pushed down, thereby rotating the syringe cap until the nubs 589 abut the vertical faces 571 of the saw-toothed projections 567. The movement of the syringe cap 527 during insertion is indicated by the arrows on Fig. 10a. At this point, the shoulder 565 prevents further insertion of the syringe cap 27 into the cavity 535.

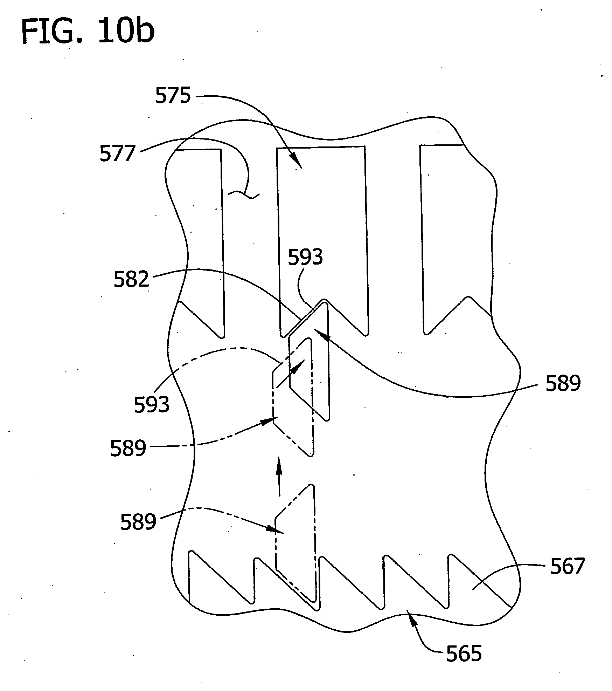

Upon release of the syringe cap 527 by the user (Fig. 10b), the spring 563 causes reverse axial movement of the syringe cap 527 outward from the cavity 535 until the nubs 589, which are no longer aligned with the channels 577 engage the inclined surfaces 582 on the bottom of the latch tabs 575. The inclined surfaces 582 exert an additional torque on the syringe cap 527, thereby further rotating the syringe cap until the nubs 589 are wedged into the notches 583 therein. The movement of the syringe cap 527 after its release is indicated by the arrows on Fig. 10b. This moves the lower surface 595 out of alignment with the inclined surface 573 of the shoulder 565 that it previously engaged and into alignment with the next inclined surface to the right (as shown in the drawings). The latch tabs 575 hold the syringe cap 527 securely in the cap retainer 531. Thus, the user may pull the syringe 107 and the needle 125 attached thereto out of the syringe cap 527 by pulling against the grip of the latch tabs 575 on the syringe cap.

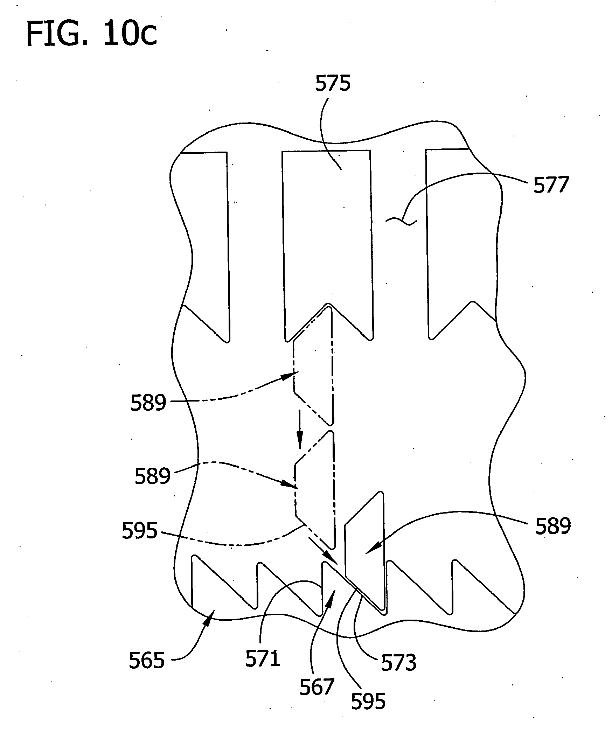

When the user is ready to recap the needle 125 (e.g., after the needle has been used to inject the radiopharmaceutical into a patient), he or she inserts the needle back into the syringe cap 527 while it is being held by the cap retainer (Fig. 10c). When the radiopharmaceutical syringe 107 bottoms out in the syringe cap 527, the syringe cap will be pushed back into the cavity 535 against the bias of the spring 563 until the nubs 589 engage inclined surfaces 573 of the saw-tooth projections 567 on the shoulder 565 that are generally aligned with the notches 583 in the latch tabs 575. The inclined surfaces 573 of the saw-toothed projections 567 exert a torque on the syringe cap 527 that results in additional rotation of the syringe cap until the nubs 589 abut the vertical faces 571 of the saw-toothed projections 567. The shoulder 565 prevents further movement of the syringe cap into the cavity, allowing the user to exert enough pressure to fully seat the radiopharmaceutical syringe in the syringe cap. Movement of the syringe cap 527 during reinsertion of the radiopharmaceutical syringe therein is indicated by the arrows on Fig. 10c.

Upon release of the radiopharmaceutical syringe 107 by the user, the spring 563 moves the syringe cap 527 in the reverse axial direction out of the cavity 535. The upper inclined surfaces 593 of the nubs 589, which have been rotated so they no longer align with the notches 583 of the latch tabs 575, engage lower corners 585 of the latch tabs. The lower corners 585 of the latch tabs 575 exert an additional torque on the syringe cap 527 resulting in additional rotation of the syringe cap until the nubs 589 are aligned with the channels 577. Once the nubs 589 are in the channels 577, the spring 563 pushes the syringe cap 527 out of the cavity 535, thereby resulting in release of the syringe cap by the cap retainer 531.

Those skilled in the art will recognize that the embodiments described above can be modified without departing from the scope of the invention. The cap retainer can be provided on either the top or bottom portion of the radiopharmaceutical pig. The radiopharmaceutical container can have virtually any shape and size. The radiopharmaceutical containers of the present invention can be modified, if necessary, to accommodate virtually any style of syringe cap. The syringe cap can either be attached to the unused radiopharmaceutical syringe when the radiopharmaceutical container arrives at the healthcare facility or it can be provided by the healthcare facility for attachment to a spent radiopharmaceutical syringe. Further, those skilled in the art will recognize that individual features of the embodiments discussed above can be combined in various ways as desirable for any particular application.

When introducing elements of the present invention or the preferred embodiments thereof, the articles "a", "an", "the", and "said" are intended to mean that there are one or more of the elements. The terms "comprising", "including", and "having" are intended to be inclusive and mean that there may be additional elements other than the listed elements. Moreover, the use of "top" and "bottom" and variations of these terms is made for convenience, but does not require any particular orientation of the components.

As various changes could be made in the above products and methods without departing from the scope of the invention, it is intended that all matter contained in the above description and shown in the accompanying drawings shall be interpreted as illustrative and not in a limiting sense.

## Claims

1. A radiation-shielding container (101) for holding a radiopharmaceutical syringe that includes a needle and a syringe cap covering the needle, the container comprising:
top and bottom portions (103,105), the top portion being releasably securable to the bottom portion for enclosing the syringe in the container, each of the top and bottom portions (103,105) comprising radiation-shielding material **characterized in that** said container further comprises;
a cap retainer (131) for selectively holding a syringe cap (127) so that the cap is held in a position in which an open end of the cap (127) is disposed for insertion of a syringe needle therein after use of the needle, wherein the cap retainer (131) is located on a top end of the top portion (103) of the container, and wherein the cap retainer (131) comprises a cavity sized and shaped for receiving a closed end part of a syringe cap (127) and holding the syringe cap so that it projects outwardly from the top end of the container (101) and presents an open end part of the syringe cap (127) for receiving a syringe needle.

2. The radiation-shielding container of claim 1 wherein the cap retainer (131) is located on an exterior surface of the container (101).

3. The container of claim 1 or claim 2 wherein the cap retainer (131) is adapted to grip a syringe cap (127) upon insertion therein so as to permit the syringe cap (127) to be pulled off a syringe needle without holding the syringe cap in the hand.

4. The container of claim 3 wherein the cap retainer (131) is further adapted to selectively release a syringe cap to permit a capped syringe to be placed in the container.

5. The container of claim 1 wherein the cavity (231) has an open end that is inside said one of the first and second portions (203,205) so that the cavity is enclosed in the container (201) when the first and second portions are secured to one another.

6. The radiation-shielding container of any preceding claim and of radiation-shielding material comprising at least one of lead, tungsten, depleted uranium, and tungsten impregnated plastic.

7. The radiation-shielding container of claim 4 wherein the cap retainer (131) comprises a cavity in the container sized and shaped for receiving a closed end of a syringe cap (127) and a gripping mechanism (53, 561) operable when the cap (127) is initially pushed into the cavity to grip and hold the cap in the cavity and operable when the cap is pushed into the cavity while being gripped by the gripping mechanism in the cavity to release the cap.

8. The radiation-shielding container of any of claims 1-7, the container further comprising a second cap retainer (231) for selectively holding a second syringe cap (127) so the second syringe cap is held in a position in which an open end of the second syringe cap is disposed for insertion of a second syringe needle therein.

9. The radiation-shielding container of any preceding claim, wherein the cap retainer (131,231) is supported by at least one of said first and second portions of the container, said at least one portion of the container being a freestanding container portion adapted for freestanding support of the cap retainer in a user accessible position for capping the needle.

10. The radiation-shielding container of claim 9, and adapted so that a force required to tip said at least one portion of the container (101,201) over when said at least one portion is supported by a surface in a freestanding position in which the cap retainer is in a user accessible position is greater than a securement force required to secure the syringe cap to the syringe to cap the needle.

11. The radiation-shielding container of claim 9, wherein said at least one portion of the container (101,201) has a weight that is greater than a securement force required to secure the syringe cap to the syringe to cap the needle.

12. The radiation-shielding container of any preceding claim, wherein the container (101,201) has a longitudinal axis and the cap retainer has a longitudinal axis generally parallel to the longitudinal axis of the container.

13. The radiation-shielding container (101,201) of any preceding claim in combination with a radiopharmaceutical syringe therein.

14. The radiation-shielding container of claim 13 in combination with a dose of a radiopharmaceutical in the radiopharmaceutical syringe.

15. A method of using the radiation-shielding container of claim 1, the method comprising:
removing a syringe cap (127) from a radiopharmaceutical syringe, wherein the removing comprises engaging the syringe cap with the cap retainer (131) so that the cap retainer holds the syringe cap such that the syringe cap projects from the top portion and presents an open end part of the syringe cap for receiving a syringe needle;
ejecting a radiopharmaceutical from the radiopharmaceutical syringe;
after the ejecting step, inserting a needle of the radiopharmaceutical syringe into the open end of the syringe cap while the syringe cap is held by the cap retainer, to re-attach the syringe cap to the radiopharmaceutical syringe.

16. The method of claim 15 wherein the engaging step comprises inserting at least a portion of the syringe cap into a cavity defined in the cap retainer, the syringe cap being gripped in the cavity.

17. The method of claim 15 or 16, further comprising, after the inserting step, enclosing the radiopharmaceutical syringe within the radiation-shielding container.

18. The method of any of claims 15-17, wherein the inserting step comprises manually inserting the needle into the cap held in the cap retainer.

19. The method of claim 18, wherein the manually inserting step is accomplished without manually holding the radiation-shielding container.

## Patentansprüche

1. Strahlenschutzbehälter (101) zum Halten einer radiopharmazeutischen Spritze, die eine Nadel und eine Nadelkappe, die die Nadel bedeckt, beinhaltet, wobei der Behälter umfasst:
obere und untere Teile (103, 105), wobei der obere Teil zum Einschließen der Spritze in dem Behälter lösbar am unteren Teil befestigt werden kann, wobei jeder der oberen und unteren Teile (103, 105) Strahlenschutzmaterial umfasst, **dadurch gekennzeichnet, dass** der Behälter weiterhin umfasst:
eine Kappenhalterung (131), um selektiv eine Spritzenkappe (127) zu halten, so dass die Kappe in einer Position gehalten wird, in der ein offenes Ende der Kappe (127) zum Einführen einer Spritzennadel nach Verwendung der Nadel bereitgestellt wird, wobei die Kappenhalterung (131) sich am oberen Ende des oberen Teils (103) des Behälters befindet, und wobei die Kappenhalterung (131) einen Hohlraum umfasst, der die Größe und Form hat, um ein geschlossenes Endstück einer Spritzenkappe (127) aufzunehmen und die Spritzenkappe zu halten, so dass sie vom oberen Ende des Behälters (101) wegragt und ein offenes Endstück der Spritzenkappe (127) zur Aufnahme einer Spritzennadel präsentiert.

2. Strahlenschutzbehälter nach Anspruch 1, wobei die Kappenhalterung (131) sich auf einer Außenfläche des Behälters (101) befindet.

3. Behälter nach Anspruch 1 oder Anspruch 2, wobei die Kappenhalterung (131) ausgelegt ist, um eine Spritzenkappe (127) beim Einführen so zu greifen, dass die Spritzenkappe (127) von einer Spritzennadel abgezogen werden kann, ohne die Spritzenkappe in der Hand zu halten.

4. Behälter nach Anspruch 3, wobei die Kappenhalterung (131) weiterhin ausgelegt ist, um selektiv eine Spritzenkappe freizugeben, so dass eine mit Kappe versehene Spritze in den Behälter platziert werden kann.

5. Behälter nach Anspruch 1, wobei der Hohlraum (231) eine offenes Ende hat, das sich innerhalb einem der ersten und zweiten Teile (203, 205) befindet, so dass der Hohlraum von dem Behälter (201) umschlossen ist, wenn die ersten und zweiten Teile aneinander befestigt sind.

6. Strahlenschutzbehälter nach einem der vorhergehenden Ansprüche und aus Strahlenschutzmaterial, umfassend wenigstens eines ausgewählt aus Blei, Wolfram, abgereichertem Uran und wolframimprägniertem Kunststoff.

7. Strahlenschutzbehälter nach Anspruch 4, wobei die Kappenhalterung (131) in dem Behälter einen Hohlraum umfasst, der die Größe und Form hat, um ein geschlossenes Ende einer Spritzenkappe (127) aufzunehmen, und einen Greifmechanismus (531, 561), der betätigt wird, wenn die Kappe (127) zu Beginn in den Hohlraum gedrückt wird, um die Kappe zu greifen und in dem Hohlraum zu halten, und betätigt wird, wenn die Kappe in den Hohlraum gedrückt wird, während sie von dem Greifmechanismus in dem Hohlraum ergriffen ist, um die Kappe freizugeben.

8. Strahlenschutzbehälter nach einem der Ansprüche 1-7, wobei der Behälter weiterhin eine zweite Kappenhalterung (231) umfasst, um selektiv eine zweite Spritzenkappe (127) zu halten, so dass die zweite Spritzenkappe in einer Position gehalten wird, in der ein offenes Ende der zweiten Spritzenkappe zum Einführen einer zweiten Spritzennadel bereitgestellt wird.

9. Strahlenschutzbehälter nach einem der vorhergehenden Ansprüche, wobei die Kappenhalterung (131, 231) von wenigstens einem der ersten und zweiten Teile des Behälters getragen wird, wobei der wenigstens eine Teil des Behälters ein freistehender Behälterteil ist, der als freistehender Träger für die Kappenhalterung in einer benutzerzugänglichen Position ausgelegt ist, um die Nadel mit einer Kappe zu versehen.

10. Strahlenschutzbehälter nach Anspruch 9, der so ausgelegt ist, dass eine Kraft, die notwendig ist, um den wenigstens einen Teil des Behälters (101, 201) umzukippen, wenn der wenigstens eine Teil von einer Fläche in einer freistehenden Position getragen wird, in der die Kappenhalterung sich in einer benutzerzugänglichen Position befindet, größer ist als eine Befestigungskraft, die notwendig ist, um die Spritzenkappe an der Spritze zu befestigen, um die Nadel zu bedecken.

11. Strahlenschutzbehälter nach Anspruch 9, wobei der wenigstens eine Teil des Behälters (101, 201) ein Gewicht hat, das größer ist als eine Befestigungskraft, die notwendig ist, um die Spritzenkappe an der Spritze zu befestigen, um die Nadel zu bedecken.

12. Strahlenschutzbehälter nach einem der vorhergehenden Ansprüche, wobei der Behälter (101, 201) eine Längsachse hat und die Kappenhalterung eine Längsachse hat, die im Allgemeinen parallel zu der Längsache des Behälters verläuft.

13. Strahlenschutzbehälter (101, 201) nach einem der vorhergehenden Ansprüche in Kombination mit einer radiopharmazeutischen Spritze darin.

14. Strahlenschutzbehälter nach Anspruch 13 in Kombination mit einer Dosis eines Radiopharmazeutikums in der radiopharmazeutischen Spritze.

15. Verfahren zur Verwendung des Strahlenschutzbehälters nach Anspruch 1, wobei das Verfahren umfasst:
Entfernen einer Spritzenkappe (127) von einer radiopharmazeutischen Spritze, wobei das Entfernen Einrasten der Spritzenkappe in die Kappenhalterung (131) umfasst, so dass die Kappenhalterung die Spritzenkappe hält, so dass die Spritzenkappe von dem oberen Teil wegragt und ein offenes Endstück der Spritzenkappe zur Aufnahme einer Spritzennadel präsentiert;
Entlassen eines Radiopharmazeutikums aus der radiopharmazeutischen Spritze;
nach dem Entlassungsschritt, Einführen einer Nadel der radiopharmazeutischen Spritze in das offene Ende der Spritzenkappe, während die Spritzenkappe von der Kappenhalterung gehalten wird, um die Spritzenkappe wieder an der radiopharmazeutischen Spritze zu befestigen.

16. Verfahren nach Anspruch 15, wobei der Einrastschritt Einführen wenigstens eines Teils der Spritzenkappe in einen in der Kappenhalterung definierten Hohlraum umfasst, wobei die Spritzenkappe in dem Hohlraum festgehalten wird.

17. Verfahren nach Anspruch 15 oder 16, weiterhin umfassend, nach dem Einführungsschritt, Einschließen der radiopharmazeutischen Spritze in dem Strahlenschutzbehälter.

18. Verfahren nach einem der Ansprüche 15-17, wobei der Einführungsschritt manuelles Einführen der Nadel in die in der Kappenhalterung gehaltenen Kappe umfasst.

19. Verfahren nach Anspruch 18, wobei der manuelle Einführungsschritt ohne manuelles Halten des Strahlenschutzbehälters durchgeführt wird.

## Revendications

1. Contenant à écran contre le rayonnement (101) destiné à contenir une seringue radiopharmaceutique qui comprend une aiguille et une coiffe de seringue couvrant l'aiguille, le contenant comprenant :
des parties supérieure et inférieure (103, 105), la partie supérieure étant fixable de façon amovible à la partie inférieure pour renfermer la seringue dans le contenant, chacune des parties supérieure et inférieure (103, 105) comprenant un matériau faisant écran au rayonnement, **caractérisé en ce que** ledit contenant comprend en outre ;
un élément de retenue de la coiffe (131) pour maintenir sélectivement une coiffe de seringue (127) de sorte que la coiffe soit maintenue dans une position dans laquelle une extrémité ouverte de la coiffe (127) est aménagée pour l'insertion d'une aiguille de seringue dans celle-ci après utilisation de l'aiguille, l'élément de retenue de la coiffe (131) étant situé sur une extrémité supérieure de la partie supérieure (103) du contenant, et l'élément de retenue de la coiffe (131) comprenant une cavité dimensionnée et formée de façon à recevoir une partie terminale fermée d'une coiffe de seringue (127) et à maintenir la coiffe de seringue de telle sorte qu'elle se projette vers l'extérieur de l'extrémité supérieure du contenant (101) et présente une partie terminale ouverte de la coiffe de seringue (127) pour recevoir une aiguille de seringue.

2. Conteneur à écran contre le rayonnement selon la revendication 1, dans lequel l'élément de retenue de la coiffe (131) est situé sur une surface extérieure du contenant (101).

3. Contenant selon la revendication 1 ou la revendication 2, dans lequel l'élément de retenue de la coiffe (131) est adapté pour saisir la coiffe de seringue (127) après insertion dans celle-ci de façon à permettre à la coiffe de seringue (127) d'être poussée de l'aiguille de la seringue sans que l'on tienne la coiffe de seringue dans la main.

4. Contenant selon la revendication 3, dans lequel l'élément de retenue de la coiffe (131) est en outre adapté de façon à libérer sélectivement une coiffe de seringue pour permettre à une seringue coiffée d'être placée dans le contenant.

5. Contenant selon la revendication 1, dans lequel la cavité (231) a une extrémité ouverte qui est à l'intérieur de l'une desdites première et deuxième parties (203, 205) de sorte que la cavité est renfermée dans le contenant (201) lorsque les première et deuxième parties sont fixées l'une à l'autre.

6. Contenant à écran contre le rayonnement selon l'une quelconque des revendications précédentes et matériau servant d'écran contre le rayonnement comprenant au moins du plomb, du tungstène, de l'uranium appauvri et du plastique imprégné de tungstène.

7. Contenant à écran contre le rayonnement selon la revendication 4, dans lequel ledit élément de retenue de la coiffe (131) comprend une cavité dans le contenant, dimensionnée et formée de façon à recevoir une extrémité fermée d'une coiffe de seringue (127) et un mécanisme de préhension (531, 561) pouvant être actionné lorsque la coiffe (127) est poussée initialement dans la cavité pour saisir et retenir la coiffe dans la cavité et pouvant être actionné lorsque la coiffe est poussée dans la cavité tout en étant saisie par le mécanisme de préhension dans la cavité pour libérer la coiffe.

8. Contenant à écran contre le rayonnement selon l'une quelconque des revendications 1 à 7, le conteneur comprenant en outre un deuxième élément de retenue de la coiffe (231) pour retenir sélectivement une deuxième coiffe de seringue (127) de telle sorte que la coiffe de seringue soit retenue dans une position dans laquelle une extrémité ouverte de la deuxième seringue est aménagée pour l'insertion d'une deuxième aiguille de seringue dans celle-ci.

9. Contenant à écran contre le rayonnement selon l'une quelconque des revendications précédentes, dans lequel l'élément de retenue de la coiffe (131, 231) est soutenu par au moins l'une desdites première et deuxième parties du conteneur, au moins ladite partie du conteneur étant une partie du conteneur autoportante adaptée pour soutenir de façon autoportante l'élément de retenue de la coiffe dans une position accessible pour un utilisateur pour coiffer l'aiguille.

10. Contenant à écran contre le rayonnement selon la revendication 9, adapté de telle sorte qu'une force nécessaire pour faire basculer au moins ladite partie du contenant (101, 201), lorsqu'au moins ladite partie est soutenue par une surface dans une position autoportante dans laquelle l'élément de retenue de la coiffe est en position accessible à un utilisateur, soit supérieure à une force de fixation nécessaire pour fixer la coiffe de seringue à la seringue pour coiffer l'aiguille.

11. Contenant à écran contre le rayonnement selon la revendication 9, dans lequel au moins une partie du conteneur (101, 201) a un poids qui est supérieur à la force de fixation nécessaire pour fixer la coiffe de seringue à la seringue pour coiffer l'aiguille.

12. Contenant à écran contre le rayonnement selon l'une quelconque des revendications précédentes, le contenant (101, 201) ayant un axe longitudinal et l'élément de retenue de la coiffe ayant un axe longitudinal généralement parallèle à l'axe longitudinal du contenant.

13. Contenant à écran contre le rayonnement (101, 201) selon l'une quelconque des revendications précédentes, en combinaison avec une seringue radiopharmaceutique dans celui-ci.

14. Contenant à écran contre le rayonnement selon la revendication 13, en combinaison avec une dose de produit radiopharmaceutique dans la seringue radiopharmaceutique.

15. Procédé d'utilisation du contenant à écran contre le rayonnement selon la revendication 1, le procédé comprenant :
le retrait d'une coiffe de seringue (127) d'une seringue radiopharmaceutique, le retrait comprenant la mise en contact de la coiffe de seringue avec l'élément de retenue de la coiffe (131) de telle sorte que l'élément de retenue de la coiffe retienne la coiffe de seringue de telle sorte que la coiffe de seringue se projette de la partie supérieure et
présente une partie d'extrémité ouverte de la coiffe de seringue pour recevoir une aiguille de seringue ;
l'éjection d'un produit radiopharmaceutique de la seringue radiopharmaceutique ;
après l'étape d'éjection, l'insertion d'une aiguille de la seringue radiopharmaceutique dans l'extrémité ouverte de la coiffe de seringue alors que la coiffe de seringue est retenue par l'élément de retenue de la coiffe, pour fixer à nouveau la coiffe de seringue à la seringue radiopharmaceutique.

16. Procédé selon la revendication 15, dans lequel l'étape de mise en prise comprend l'insertion d'au moins une partie de la coiffe de seringue dans une cavité définie dans l'élément de retenue de la coiffe, la coiffe de seringue pouvant être saisie dans la cavité.

17. Procédé selon la revendication 15 ou 16, comprenant en outre, après l'étape d'insertion, l'insertion de la seringue radiopharmaceutique dans le contenant à écran contre le rayonnement.

18. Procédé selon l'une quelconque des revendications 15 à 17, dans lequel l'étape d'insertion comprend l'insertion manuelle de l'aiguille dans la coiffe maintenue dans l'élément de retenue de la coiffe.

19. Procédé selon la revendication 18, dans lequel l'étape d'insertion manuelle est réalisée sans tenir manuellement le contenant à écran contre le rayonnement.
